Europäisches Patentamt

European Patent Office

Office européen des brevets

(19)

(11) Publication number: **0 290 012**

**A1**

(12) # EUROPEAN PATENT APPLICATION

(21) Application number: **88107174.0**

(22) Date of filing: **04.05.88**

(51) Int. Cl.⁴: **C12N 15/00 , A61K 37/00 , C12P 21/00 , C07K 7/00**

(30) Priority: **04.05.87 US 46846**
**30.10.87 US 115776**

(43) Date of publication of application:
**09.11.88 Bulletin 88/45**

(84) Designated Contracting States:
**AT BE CH DE ES FR GB GR IT LI LU NL SE**

(71) Applicant: **ONCOGEN**
**3005 First Avenue**
**Seattle, WA 98121(US)**

(72) Inventor: **Marquardt, Hans**
**9222 S.E. 46th Street**
**Mercer Island Washington 98040(US)**
Inventor: **Ikeda, Tatsuhiko**
**2-1-12, Hoshigaoka**
**Tarumi-ku Kobe 655(JP)**
Inventor: **Lioubin, Mario N.**
**4130 156th Lane, S.E.**
**Bellevue Washington 98006(US)**

(74) Representative: **Kinzebach, Werner, Dr. et al**
**Patentanwälte Reitstötter, Kinzebach und**
**Partner Sternwartstrasse 4 Postfach 86 06 49**
**D-8000 München 86(DE)**

(54) TGF-B2 and novel compositions having anti-neoplastic activity.

(57) A novel transforming growth factor designated TGF-$\beta 2$, is provided for inhibiting growth of cells, particularly tumor cells. A means for inducing production of TGF-$\beta 2$ as a method for inhibiting proliferation of responsive cells is also provided. Production of TGF-$\beta 2$ may also be used as a probe to identify cells sensitive to the anti-proliferative effects of TGF-$\beta 2$ or inducing compounds.

EP 0 290 012 A1

# TGF-$\beta$2 AND NOVEL COMPOSITIONS HAVING ANTI-NEOPLASTIC ACTIVITY

## Technical Field

Cell growth regulatory compositions are disclosed, where the compounds work independently and provide for synergistic effects in combination.

## Background

The ability to control tumor cell growth without serious side effects to the host organism has long been a goal in the continuing search for improved methods for treating cancer. Drugs or naturally occurring compounds usually have a broad range of activities towards different cells. Moreover, at concentrations which may be effective in inhibiting the growth of neoplastic cells, these compounds frequently demonstrate undesired effects on normal cells. Of particular interest therefore are compounds which selectively inhibit the growth of neoplastic cells while not inhibiting the growth of surrounding or other normal tissue.

Examples of such compounds are peptides such as the interferons and lymphocyte-derived tumor necrosis factor-$\beta$ (TNF-$\beta$) as well as TNF-$\alpha$, derived from myelocytic cells, which display cytostatic and/or cytocidal activity against transformed cell lines, but do not affect normal cells. However, even when those compounds such as the interferons which show inhibitory activity primarily towards tumor cells, not all tumor cells respond to the compound. In addition, reliable methods have not been developed to identify tumors which have a high probability of responding to a given compound. Furthermore, there may be a fine line between a therapeutic dose of a given compound and that which is physiologically unacceptable or toxic.

The family of peptides known as transforming growth factor type $\beta$ (TGF-$\beta$) regulate cell growth and differentiation. These growth regulatory polypeptides can both stimulate (TGF-$\beta$1 and TGF-$\beta$2) and inhibit (TGF-$\beta$1 and TGF-$\beta$2) cell proliferation, depending largely on the cell type. TGF-$\beta$1 and TGF-$\beta$2 have a common anti-proliferative effect on epithelial cell growth as well as a high degree of receptor cross-reactivity. TGF-$\beta$1 is produced by carcinoma, sarcoma, and melanoma cells. Production of TGF-$\beta$1 appears to be constitutive. In contrast, production of TGF-$\beta$2 is restricted to carcinoma cell lines of different origins and appears to be hormonally induced.

There is therefore an interest in being able to identify those tumor cells which may respond to a given anti-proliferative compound. Furthermore, since these anti-proliferative compounds are produced by the tumor cells it is also of interest to be able to both identify and monitor the effective dose of compounds which may release endogenous anti-proliferative compounds.

## Relevant Literature

Reversal of anti-estrogen induction of TGF-$\beta$ by estrogen in MCF-7 cells has been reported by Knabbe et al., Cell (1987) 48:417-428. Purification and initial characterization of human TGF-$\beta$2 from an adenocarcinoma cell line has been described by Ikeda et al., Biochemistry (1987) 26:2406-2410 and from porcine blood platelets by Cheifetz et al., Cell (1987) 48:409-415. A review of the family of compounds under the designation TGF-$\beta$ is provided by Sporn et al., Science (1986) 233:532-534. The structure and functional relationship of CIF-B (cartilage-inducing factor B) to TGF-$\beta$ is demonstrated by Seyedin et al., J. Biol. Chem. (1987) 262:1946-1949. See also Seydin et al., Proc. Natl. Acad. Sci. USA (1985) 82:2267-2271.

## SUMMARY OF THE INVENTION

A novel transforming growth factor-$\beta$, designated TGF-$\beta$2, is provided, which factor is available from tamoxifen-supplemented medium conditioned by human prostatic adenocarcinoma cells or human breast carcinoma cells. The factor finds use in inhibiting proliferation of neoplastic cells and as a probe for identifying cells which may be sensitive to the inhibitory effects of the factor. Detection of increased production of the factor following anti-estrogen administration may be indicative of cells which are growth-inhibited by the anti-estrogen.

## DESCRIPTION OF THE SPECIFIC EMBODIMENTS

In accordance with the subject invention, novel anti-neoplastic compositions comprising TGF-$\beta$2 and fragments thereof, as well as methods of preparation and use, are provided. TGF-$\beta$2 is characterized as being produced by estrogen-responsive carcinoma cells in the presence of the anti-estrogen 1-p-$\beta$-dimethylaminoethoxyphenyl-trans-1,2-diphenylbut-1-ene (Tamoxifen). TGF-$\beta$2 is further characterized as having a molecular weight of about 24,000 as determined by analytical SDS-PAGE gel electrophoresis and has the following amino acid sequence:

```
              5           10          15
A-L-D-A-A-Y-C-F-R-N-V-Q-D-N-C
              20          25          30
C-L-R-P-L-Y-I-D-F-K-R-D-L-G-W
              35          40          45
K-W-I-H-E-P-K-G-Y-N-A-N-F-C-A
              50          55          60
G-A-C-P-Y-L-W-S-S-D-T-Q-H-S-R
              65          70          75
V-L-S-L-Y-N-T-I-N-P-E-A-S-A-S
              80          85          90
P-C-C-V-S-Q-D-L-E-P-L-T-I-L-Y
              95         100         105
Y-I-G-K-T-P-K-I-E-Q-L-S-N-M-I
             110
V-K-S-C-K-C-S
```

The above sequence is the human sequence. Besides the above sequence, which is a single sub-unit of the dimer, the dimer is also induced, as well as fragments thereof. Of particular interest is an amino acid sequence of at least about 8 amino acids in the region from amino acids 1 to 20, more particularly 4 to 15 and within this region, 9 to 14. Also of interest is the C-terminal sequence of amino acids 85 to 112, more particularly 90 to 112.

TGF-$\beta$2 may be obtained in a variety of ways. It is available from naturally occurring sources, particularly growth medium supplemented with an anti-estrogen and conditioned by estrogen responsive tumor cells. Various carcinoma cells, especially carcinoma cells whose growth rate is affected by estrogens and/or anti-estrogens, may be used as a source for TGF-$\beta$2. The cells can be grown in growth medium containing phenol red, which has weak estrogenic activity or in phenol red-free growth medium supplemented with an estrogen such as 17$\beta$-estradiol. For increased production of TGF-$\beta$2, the medium can be additionally supplemented with an anti-estrogen such as Tamoxifen.

TGF-$\beta$2 can be purified so as to be substantially free of cellular components employing various purification techniques. These techniques can include solvent extraction, gel permeation chromatography, reversed phase-HPLC, electrophoresis, or the like. For active fragments, various synthetic techniques may be employed, where the polypeptide will be synthesized on a solid support. A number of commercial synthesizers are available and may be used to advantage, for example, from Smith Kline, Beckman, Applied Biosystems, etc.

TGF-β2 or fragments thereof also can be prepared by employing recombinant techniques. The amino acid sequence may be used to design a probe and a cDNA library or genomic library from stimulated or unstimulated prostatic carcinoma (PC3) cells screened for hybridizing sequences. To enhance the likelihood of identifying the correct sequence, a cDNA library from unstimulated prostatic adenocarcinoma cells or other related cell line which does not produce TGF-β2 may be used to cross-hybridize. Those sequences from the PC3 cells which do not hybridize will be concentrated as to the desired sequence. Sequences which hybridize to the probe and do not cross-hybridize with cDNA from cells which do not produce TGF-β2 may be screened by using Xenopus oocytes. An assay for the expression of TGF-β2 using restriction fragments inserted into a prokaryotic expression vector such as λgt-11 then screening with antibodies for TGF-β2 to detect a cross-reactive peptide fragment or the like can be used. Once the fragment containing the sequence encoding TGF-β2 has been identified, the fragment may be manipulated by resec tion, primer repair, in vitro mutagenesis, restriction endonuclease digestion, or the like, to provide a different 5′ transcriptional and, as appropriate, translational initiation region and optionally a different 3′ transcriptional and, as appropriate, translational termination region. Various expression vectors into which the open reading frame encoding TGF-β2 may be inserted are commercially available or described in the literature. The vector may be transformed into an appropriate host for expression. Prokaryotic expression hosts include E. coli and B. subtilis; eukaryotic hosts include S. cerevisiae, CHO cells, monkey kidney cells, streptomyces, etc.

The subject compositions may be modified by truncating at the N-or C-terminus where up to about 10 amino acids may be removed. However, for biological activity the C-terminus should be retained. In addition, up to about 5 mole percent of non-conservative mutations may be made and up to 10 mole percent of conservative mutations may be made. For conservative substitutions, the groups may be broken down into non-polar aliphatic amino acids (G,A,P,V,I,L), polar aliphatic amino acids (C,S,T,M,N,Q), aromatic amino acids (F,H,W,Y), basic amino acids (K,R), and acidic amino acids (D,F).

The TGF-β2 or fragments thereof may be used as an anti-neoplastic compound. The compound is provided having a relative specific activity (see Table 1, below) of about 45 units/ng, usually at least about 60 units/ng, preferably at least about 70 units/ng, and up to complete purity.

For use in vivo, the subject peptides may be administered in a variety of ways, by injection, by infusion, topically, parenterally, or the like. Administration may be in any physiologically acceptable carrier, such as sterilized water, phosphate buffered saline, saline, aqueous ethanol, etc.

The subject peptides may be formulated in a variety of ways, including in the lumen of liposomes, particularly where the liposomes may be bound to homing molecules (e.g., antibodies) which target for a particular neoplastic cell in degradable particle matrices, or the like.

Other components may be included in the formulation such as buffers, stabilizers, surfactants, biocides, etc. These components have found extensive exemplification in the literature and need not be described in particular here.

TGF-β2 and fragments thereof find a wide variety of uses. The subject peptides may be used either alone or with at least one other anti-proliferative compound, for example an interferon, TGF-β1, tumor necrosis factor-β, tumor nercrosis factor-α, etc., in the treatment of hormonally responsive carcinomas which may affect a wide variety of organs, such as the lungs, breast, prostate, colon, etc. Hormonally responsive carcinomas can also be treated by inducing production of TGF-β2 in the carcinoma cells. Inducers include estrogens such as 17-β estradiol; compounds with estrogenic activity; and compounds with anti-estrogenic activity such as Tamoxifen. Any combination of these compounds may also be used as an inducer.

The most effective concentration of TGF-β2 for inhibiting proliferation of a given cell may be determined by adding various concentrations of TGF-β2 to the tumor cell of interest and monitoring the amount of inhibition of cell proliferation. The most effective concentration of individual inducers and/or combinations of inducers may be determined by monitoring the production of TGF-β2 in the carcinoma cells. The type and amount of TGF produced can be determined by reversed phase HPLC.

Besides being used for their growth inhibiting properties, TGF-β2 and fragments thereof may be used in diagnostics. Thus, TGF-β2 or fragments thereof may be joined to a label, such as radioisotope, enzyme, fluorescer, chemiluminescer, enzyme fragment, particle, etc. A wide variety of diagnostic assays are known, see, for example, U.S. Patent Nos. 3,791,932; 3,817,837; 4,134,792; 3,996,345. The subject compounds also may be used for the production of antibodies, either polyclonal or monoclonal, where the antibodies may be used for detection of TGF-β2 in diagnostic assays, for studying the effects of TGF-β2 on cells in culture, for detecting the presence of TGF-β2 in conditioned media, and the like.

Examples of diagnostic uses for TGF-$\beta$2 include titrating the number of receptors for TGF-$\beta$2 on a cell. Identification of receptors for TGF-$\beta$2 is an indication of potential responsiveness of the cell to the growth inhibitory effects of TGF-$\beta$2. TGF-$\beta$2 and/or antibodies thereto also may be used in competitive assays for detection of TGF-$\beta$2 in media, particularly physiological media. Detection of TGF-$\beta$2, especially an increase in TGF-$\beta$2 following addition of an estrogen and/or an anti-estrogen to the cell to be tested, can be used as an indication of the potential responsiveness of a cell to the effects of the inducing compound.

The following examples are offered by way of illustration and not by way of limitation.

## EXPERIMENTAL

### Example 1

#### Isolation of TGF-$\beta$2

##### A. Source of TGF-$\beta$2

TGF-$\beta$2 was purified from Tamoxifen-supplemented medium conditioned by a line of human prostatic adenocarcinoma cells, PC-3 (Kaighn et al., Invest. Urol. (1979) 17:16-23, derived from a bone metastasis. The PC-3 line was obtained from the American Type Culture Collection (Rockville, MD). Cells were grown to confluence on 850 sq. cm plastic roller bottles (Corning 25140) in 50 ml of growth medium which comprises F-12 HAM (Sigma, St. Louis, MO) and Dulbecco's modified Eagle's medium (1:1), supplemented with 10% heat-inactivated fetal bovine serum (FBS). The monolayers were washed three times with phosphate-buffered saline. The medium was discarded and replaced with 50 ml of fresh serum-free medium supplemented with Tamoxifen dissolved in dimethylsulfoxide to reach a final concentration of $1.4 \times 10^{-5}$ M. Conditioned medium was collected every 48 h for a 6-day period by decantation, clarified by low-speed centrifugation at 1000 x g for 15 min, and passed through a filter with a pore size of 0.45 $\mu$m (Nalgene, Rochester, NY). Aprotinin (3.6 mg/l) and phenylmethanesulfonyl fluoride (10 mg/l) were added to the filtrate.

##### B. Batch Adsorption on Methylsilyl-Controlled Pore Glass (MS-CpG)

PC-3-conditioned medium was acidified to pH 4.0 with concentrated trifluoroacetic acid (TFA). In the batch adsorption procedure, 30 g of MS-CpG (40 $\mu$m, Sepralyte, Analytichem International, Harbor City, CA), previously washed with 100% acetonitrile containing 0.1% TFA, were suspended in 1.6 l of acidified conditioned medium in a vessel equipped with a microcarrier magnetic stirrer (Bellco Glass, Vineland, NJ). The mixture was stirred for 1 hour at 4°C. The beads were resuspended in 10 volumes of 0.1% TFA and transferred to a column (5.0 x 3.2 cm) for elution. The column was washed with 200 ml of 0.1% TFA containing 30% acetonitrile and 0.2 M NaCl and batch eluted with 120 ml of 50% acetonitrile, 0.2 M NaCl and 0.1% TFA. The eluate containing TGF-$\beta$2 was dialyzed for 60 h against 0.1 M acetic acid, concentrated by lyophilization and reconstituted in 0.1% TFA containing 40% acetonitrile. Typically, about 50 mg of protein was recovered per preparation with a reduction in volume of about 8-fold and with a yield close to 90% of the initial growth-inhibitory activity.

##### C. Gel Permeation Chromatography Using Bio-Sil TSK-250

The supernatant containing TGF-$\beta$2 activity was further purified by gel permeation chromatography on a column (21.5 x 600 mm) of Bio-Sil TSK-250 (Bio Rad Laboratories, Richmond, CA). The column was equilibrated with 0.1% TFA containing 40% acetonitrile at 2 ml/min, at 22°C. 4 ml fractions were collected. Recovery of the initial growth inhibitory activity was 83%. One peak of activity was found with an $M_r = 14,000$ (ribonuclease A; Mr 13,700).

## D. Reversed-Phase High Performance Liquid Chromatography

hTGF-β2-containing fractions were pooled to provide 2.3 mg protein, diluted 2-fold with 0.05% TFA, and further purified by reversed-phase HPLC. All separations were performed on a μBondapak $C_{18}$ column (10 μm particle size, 3.9 x 300 mm, Waters Associates, Milford, MA). A linear acetonitrile gradient composed of 0.05% TFA in water as starting buffer and 0.045% of TFA in acetonitrile as limit buffer was used. The column was operated at a flow rate of 0.2 ml/min at 22°C. The eluant was collected in 1 ml portions. Growth-inhibitory activity of individual fractions was determined. hTGF-β2 was well separated from the bulk of contaminating protein which eluted at lower and higher concentrations of organic solvent.

Fractions N52-58 were pooled, diluted 2-fold with 0.05% TFA, and taken for rechromatography on the same column, previously equilibrated with 0.05% TFA in water. The eluant was a linear 1-propanol gradient containing 0.035% TFA at 0.2 ml/min at 22°C. The eluant was collected in 1 ml fractions. A well-defined peak of activity was separated from the bulk of UV-absorbing material. The growth-inhibitory activity co-purified with a distinct absorbance peak at 20.8% 1-propanol. Fractions from about 245-275 min were pooled and further analyzed. The purification of hTGF-β2 was approximately 15,000-fold with a yield of 74% of the initial total growth-inhibitory activity. The overall recovery of hTGF-β2 per step was 89-96%. The highest specific activity observed was 74 x 10⁶ units/mg, which gives half-maximal activity in the range of 5 x 10⁻¹² M. A summary of the purification scheme is shown in Table 1.

## E. Protein Determination

Total protein was determined (Bradford, Anal. Biochem. (1976) 72:248-254) using bovine serum albumin as a standard. Protein was also determined by UV-absorbance at 215 nm comparing peak areas with the peak area of a known amount of bovine insulin.

## Table 1

### Purification of hTGF-β2 From Conditioned Medium of Human Prostatic Adenocarcinoma Cells, PC-3

| Purification Step | Volume ml | Protein Recovered mg | hTGF-β2 Activity Recovered Units* | Relative Specific Activity Units/µg | Degree of Purification -fold | Recovery % |
|---|---|---|---|---|---|---|
| 1. PC-3-conditioned medium | 1600 | 686.0 | $3.5 \times 10^6$ | 5.1 | 1 | 100 |
| 2. Eluate from MS-CpG | 220 | 50.0 | $3.1 \times 10^6$ | 62.0 | 12 | 89 |
| 3. Bio-Sil TSK-250 | 12 | 2.3 | $2.9 \times 10^6$ | 1260.0 | 250 | 83 |
| 4. µBondapak $C_{18}$ (acetonitrile) | 7 | 0.340 | $2.8 \times 10^6$ | 8240.0 | 1620 | 80 |
| 5. µBondapak $C_{18}$ (1-propanol) | 6 | 0.035 | $2.6 \times 10^6$ | 74300.0 | 14600 | 74 |

* One unit of activity was defined as the amount of TGF-β2 required to give 50% maximal response in a growth inhibition assay (p. 19).

Example 2

Characterization of hTGF-β2

A. NaDodSO₄-Polyacrylamide Gel Electrophoresis

The purity of the final hTGF-β2 preparation was determined by analytical NaDodSO₄-polyacrylamide gel electrophoresis and compared with TGF-β1 isolated from bovine spleen. NaDodSO₄-polyacrylamide gel electrophoresis was performed as described (Laemmli, Nature (1970) 277: 680-685). A 12-20% acrylamide gradient slab (140 x 120 x 0.75 mm) was prepared with a 5% stacking gel. Molecular weight standards were ovalbumin ($M_r$ = 43,000), α-chymotrypsinogen ($M_r$ = 25,700), β-lactoglobulin ($M_r$ = 18,400), lysozyme ($M_r$ = 14,300), and bovine trypsin inhibitor ($M_r$ = 6,200). After electrophoresis, gels were fixed in 40% methanol, 10% acetic acid overnight, washed in 10% methanol, 5% acetic acid, for 2 h, and stained with silver (Merril et al., Science (1981) 211:1437-1438. One major polypeptide band, with an $M_r$ = 24,000, was observed under nonreducing conditions. When samples were electrophoresed under reducing conditions, the polypeptide stained as a single band at $M_r$ = 13,000.

B. Peptide Purification

For amino-terminal sequence analysis, hTGF-β2 (8 μg) was reduced with dithiothreitol (20 mM) in 100 μl of 0.4 M Tris HCl containing 6 M guanidine-HCl, 0.1% Na₂EDTA, pH 8.5, for 2 h at 50°C and subsequently S-pyridineethylated with 4-vinylpyridine (100 mM) for 4 h at 22°C. The reaction mixture was acidified to pH 2.0 with 20% TFA and desalted on an RP-300 column (2.1 x 30 mm, Applied Biosystems, Foster City, CA). The concentration of acetonitrile was increased linearly (1%/min) during 1 h at a flow rate of 100 μl/min, at 35°C. One symmetrical polypeptide peak was eluted with a gradient of aqueous acetonitrile containing 0.085% TFA.

Endoproteinase Lys-C peptides (38-94; 98-107; 108-110), trypsin peptides (61-94), and S. aureus V8 protease peptides (72-99) were purified by reversed-phase HPLC on an RP-300 column (2.1 x 30 mm). The elution of peptides was achieved with a 2 h linear gradient of 0.1% TFA in water to 60% acetonitrile containing 0.085% TFA at a flow rate of 50 μl/min, at 35°C. UV-absorbing material was monitored at 215 nm. Peptides were collected manually.

C. Amino acid analysis

Dried samples were gas-phase hydrolyzed with constant boiling HCl (Pierce) containing 5% thioglycolic acid (Sigma) under reduced pressure and flushed with N₂ in a Teflon-sealed glass hydrolysis bulb (Pierce) at 105°C for 24 h. The hydrolysates were dried in a Speedvac centrifugal concentrator (Savant Instruments) and derivatized with phenylisothiocyanate (Pierce) for 20 min at room temperature. Phenylthiocarbamyl amino acid derivatives were analyzed by rpHPLC on a PICO TAG column ( 39 x 150 mm; Waters Associates) with a sodium acetate-triethylamine buffer, pH 5.80/acetonitrile gradient, at 37°C, using essentially the procedure outlined by Waters Associates. Data collection and reduction were performed on an 840 Data Module (Waters Associates).

## D. Amino Acid Sequence Analysis

Sequencer reagents were obtained from Applied Biosystems; solvents for rpHPLC were from Burdick and Jackson. CNBr was from Kodak; 4-vinylpyridine was from Aldrich Chemical Co.; S-$\beta$(4-pyridylethyl)-cysteine was from Sigma; all other chemicals were reagent grade. Endoproteinase Lys-C was from Boehringer Mannheim; Staphylococcus aureus V8 protease was obtained from Miles Laboratories; L-(tosylamido-2-phenyl)ethyl chloromethyl ketone-treated trypsin was from Worthington.

Automated sequence analysis of S-pyridine-ethylated TGF-$\beta$2 was performed on a model 470A amino acid sequencer (Applied Biosystems) using the 03RPTH program. 3.0 mg of BioBrene Plus (Applied Biosystems) were applied and subjected to three precycles of Edman degradation prior to sample application. Conversion of the thiazolinone derivatives to phenylthiohydantoin amino acids was carried out using 25% TFA. Phenylthiohydantoin amino acid derivatives were separated by reversed-phase HPLC on a PTH-$C_{18}$ column (2.1 x 220 mm, Applied Biosystems) with a sodium acetate buffer containing 3.5% tetrahydrofuran/acetonitrile gradient (Hunkapiller & Hood, Science (1983) 219:650-659), online, on a model 120A PTH Analyzer (Applied Biosystems).

### a. Enzymatic cleavages.

Selected peptides obtained by cleavage with endoproteinase Lys-C (K), with trypsin (T), and with S. aureus V8 protease (E) are indicated in Table 2, below. S-pyridylethylated TGF-$\beta$2 (250 pmol) was digested with the endoproteinase Lys-C. The presence of Na dodecylsulfate was required to completely solubilize the modified TGF-$\beta$2 derivative. Cleavage with endoproteinase Lys-C was done in 40 $\mu$l of 0.1 M Tris-acetic acid buffer, pH 8.0, at 37°C for 24 h. The enzyme/substrate ratio was 1 to 10 (w/w). S. aureus V8 protease cleavage was done in a similar manner at an enzyme-substrate ratio of 1 to 10 at 37°C for 20 h. Trypsin digestion of peptide K 38-94 was done in 40 $\mu$l of 0.1 M Tris-acetic acid buffer, pH 8.0, containing 30% acetonitrile, at 37°C for 20 h. The enzymatic digests were acidified with 20% TFA to pH 2.0 and separated by rpHPLC.

Aliquots of peptide-containing fractions were taken for sequence analysis. The complete sequences of K 98-107 and K 108-110 were determined, all containing a carboxyl-terminal lysine, consistent with the enzyme specificity. Peptide K 111-112 was not retained by the RP-300 column. No attempt was made to sequence the peptide.

Fractions from about 76-81 min, containing K 38-94, and possibly partially cleaved peptides with the same amino-terminal sequence, were pooled, dried, and subfragmented with trypsin. The digest was acidified to pH 2.0 with TFA and the peptides separated by rpHPLC. The amino-terminal sequence of peptide T 61-94 was determined, which allowed assignment of the first 31 residues from position 61 through 91 of TGF-$\beta$2.

For cleavage at the carboxyl of glutamic acid, S-pyridylethylated TGF-$\beta$2 (200 pmol) was digested with S. aureus V8 protease, at pH 8.0 in the presence of 0.1% Na dodecylsulfate. The enzyme digest was acidified and peptides purified by rpHPLC. Fractions from about 77-81 min were diluted 2-fold with 0.1% TFA and rechromatographed on the same column using the same gradient conditions. Peptide E 72-99 of TGF-$\beta$2 was subjected to automated Edman degradation and was sequenced in its entirety, containing a carboxyl-terminal glutamic acid which is consistent with the enzyme specificity.

### b. Chemical cleavage.

Selected peptides obtained by cleavage with CNBr (CB) are indicated in Table 2, below. For CNBr cleavage at methionyl residues, 300 pmol of S-pyridylethylated TGF-$\beta$2 was applied onto the glass fiber sample filter of the gas-phase sequencer. The filter was saturated with 30 $\mu$l of a solution containing 15 mg CNBr in 100 $\mu$l of 70% (v/v) formic acid and then placed in a vacuum dessicator and left over CNBr/70% formic acid vapor for 20 h at room temperature. (March et al., Nature (1985) 315:641-647; Simpson and Nice, Biochemistry Internat. (1984) 8:787-791). The sample was then air-dried at 44°C for 20 min.

Sequence analysis of the mixture of peptides CB1-104 and CB105-112 confirmed the assignments made for positions 105 through 110 in the amino-terminal Edman degradation of K 98-107 and K 108-110,

9

and extended the known structure of TGF-β2 to position 112. Peptide C3105-112 did not contain a carboxyl-terminal homoserine and was thus assumed to be the carboxyl-terminal peptide of TGF-β2. Cleavage (approximately 5% of the input TGF-β2) at tryptophan 32 and 52 with excess CNBr (Ozols and Gerard. J. Biol. Chem. (1977) 252:5986-5989) yielded 2 minor identifiable sequences.

A comparison of the amino acid sequences of TGF-β2 and TGF-β1 is provided in Table 2 (below). Selected fragments of S-pyridylethylated hTGF-β2 for Edman degradation are also indicated. Peptides K, T, E, and CB are those obtained from cleavage with endoproteinase Lys-C, trypsin, S. aureus V8 protease and cyanogen bromide, respectively.

### Table 2
### Comparison of Amino Acid Sequence
### of hTGF-β2 and hTGF-β1

```
                1       5         10        15        20
hTGF-β1    A  L  D  T  N  Y  C  F  S  S  T  E  K  N  C  C  V  R  Q  L
hTGF-β2    A  L  D  A  A  Y  C  F  R  N  V  Q  D  N  C  C  L  R  P  L

                        25        30        35        40
hTGF-β1    Y  I  D  F  R  K  D  L  G  W  K  W  I  H  E  P  K  G  Y  H
hTGF-β2    Y  I  D  F  K  R  D  L  G  W  K  W  I  H  E  P  K  G  Y  N
                                                         ⌐————

                        45        50        55        60
hTGF-β1    A  N  F  C  L  G  P  C  P  Y  I  W  S  L  D  T  Q  Y  S  K
hTGF-β2    A  N  F  C  A  G  A  C  P  Y  L  W  S  S  D  T  Q  H  S  R
                              ————————K38-94————————

                        65        70        75        80
hTGF-β1    V  L  A  L  Y  N  Q  H  N  P  G  A  S  A  A  P  C  C  V  P
hTGF-β2    V  L  S  L  Y  N  T  I  N  P  E  A  S  A  S  P  C  C  V  S
           ⌐————————————————T61-94————————————————
                                           ⌐————————

                        85        90        95        100
hTGF-β1    Q  A  L  E  P  L  P  I  V  Y  Y  V  G  R  K  P  K  V  E  Q
hTGF-β2    Q  D  L  E  P  L  T  I  L  Y  Y  I  G  K  T  P  K  I  E  Q
           ——————————————————E72-99——————————
                                               ⌐————————

                        105          110
hTGF-β1    L  S  N  M  I  V  R  S  C  K  C  S
hTGF-β2    L  S  N  M  I  V  K  S  C  K  C  S
           ———K98-107——— ⌐————
                              K108-110
               ⌐——CB105-112——
```

Example 3

Production of TGF-β1 and TGF-β2 in Carcinoma Cells

A. Growth Inhibition Assay

Mv1Lu mink lung epithelial cells (CC1-64, American Type Culture Collection) were subcultured on flat-bottomed 96-well tissue culture plates (Costar 3596, Cambridge MA) in 50 $\mu$l of complete Dulbecco's modified Eagle's medium, supplemented with 10% heat-inactivated FBS, at $3.5 \times 10^3$ cells/well. Aliquots from column fractions to be assayed for growth inhibition were diluted in complete medium and assayed in triplicate with 50 $\mu$l of the diluted sample added to each well. The cells were incubated for 72 h at 37°C in a humidified 5% $CO_2$-95% air atmosphere. Each well was then incubated for 24 h with 100 $\mu$l of complete medium containing 5-[$^{125}$I]-iodo-2'-deoxyuridine ([$^{125}$-I]-IdU) 0.05 $\mu$Ci/well (Amersham IM.355V). The monolayers were washed with phosphate-buffered saline, fixed in 95% methanol, and the [$^{125}$I]-IdU incorporated by the cells solubilized with 200 $\mu$l of 1N NaOH. The amount of cell growth was measured by the amount of [$^{125}$I]-IdU incorporated into the DNA of actively growing cells. One unit of activity was defined as the amount of TGF-β1 and/or TGF-β2 required to give 50% maximal response in the above assay.

B. Isolation of TGF-β1 and TGF-β2 From Cultured Tumor Cells

Cells were grown in 150 cm² plastic flasks in 50 ml growth medium supplemented with 10% heat-inactivated FBS. For preparation of conditioned medium, cells were grown to confluence in 850 cm² plastic roller bottles in 100 ml of growth medium and 10% FBS, except for PC-3 and MCF-7 cells, which were grown in growth medium supplemented with 10% dextran-coated charcoal-treated FBS to remove endogenous steroids. The monolayers were washed with phosphate-buffered saline and the medium replaced with 100 ml of fresh growth medium without serum. Conditioned medium was collected at 48 h and 96 h, and concentrated by adding ammonium sulfate (277 g/l). The solution was stirred overnight at 4°C and centrifuged at 20,000 x g for 30 min. The pellet was reconstituted in 3 ml of 0.1% trifluoroacetic acid (TFA) in water containing 40% acetonitrile for subsequent gel permeation chromatography on a Bio-Sil TSK-250 column (BioRad Laboratories 155-0201) equilibrated wtih 0.1% TFA containing 40% acetonitrile, and reversed-phase HPLC on a $\mu$Bondapak $C_{18}$ column (Waters Associates 27324) equilibrated with 0.05% TFA in water as described. The $C_{18}$ column was eluted with a linear 6-h gradient of 17-27% 1-propanol containing 0.035% TFA at a flow rate of 0.2 ml min$^{-1}$. Fractions (1 ml) were collected and assayed for growth-inhibitory activity on Mv1Lu cells as described above. The $C_{18}$ column was calibrated with TGF-β1 from spleen and TGF-β2 from prostate carcinoma cells. PC-3, MCF-7, A673, and Mv1Lu cells were obtained from the American Type Culture Collection; L2981, C3347, and M2669 cell lines have been described and were obtained from Dr. Ingegerd Hellstrom, Oncogen, Seattle.

11

## Table 3

## Production of TGF-β1 and TGF-β2

## by Cultured Tumor Cells

| Cell Line+ | TGF-β2 (Activity** eluted at 20.5% 1-propanol) | TGF-β1 (Activity eluted at 22% 1-propanol) |
|---|---|---|
| PC-3 | 10,000 | 1,000 |
| L2981 | 9,500 | 7,500 |
| MCF-7 | 2,900 | 1,000 |
| C3347 | 4,000 | 5,000 |
| M2669 | ND* | 10,000 |
| A673 | ND* | 12,000 |

\*   Not detectable

+   PC-3, human prostatic carcinoma; L2981, human lung carcinoma; MCF-7, human breast carcinoma; C3347, colon carcinoma; M2669, human melanoma; A673, human rhabdomyosarcoma.

\*\* One unit of activity was defined as the amount of TGFβ1 and/or TGF-β2 required to give 50% maximal response in a growth inhibition assay (p. 19).

Example 4

Hormonal Control of TGF-β1 and TGF-β2

PC-3 cells were grown to confluence in roller bottles as described above (Example 3). The monolayers were washed and the medium replaced with 100 ml of fresh phenol red-containing growth medium (see Example 3B), with growth medium containing $1.4 \times 10^{-5}$M Tamoxifen, or with phenol red-free growth medium containing various concentrations of 17β-estradiol or $1.4 \times 10^{-5}$M Tamoxifen. Tamoxifen and 17β-estradiol stock solutions were prepared in dimethylsulfoxide. The final concentration of dimethylsulfoxide in the culture medium was 0.1% (v/v). Conditioned serum-free medium (200 ml) was collected and processed as described above (Example 3). The total growth-inhibitory activity was determined and purified by gel permeation chromatography and reversed-phase HPLC.

## Table 4

### Hormonal Control of TGF-β1 and

### TGF-β2 Production by PC-3 Cells

| Treatment | Activity (units) | Activity Recovered (units) | | Induction of TGF-β2 (x-fold) |
|---|---|---|---|---|
| | | TGF-β1 | TGF-β2 | |
| Phenol red-free medium +1.4 x $10^{-5}$M TAM | 5,300 | 4,200 | 500 | 1 |
| Phenol red-free medium +1 x $10^{-9}$M $E_2$ | 4,800 | NT* | NT* | - |
| Phenol red-free medium +1 x $10^{-7}$M $E_2$ | 46,700 | 4,900 | 36,600 | 73 |
| Phenol red-free medium +1 x $10^{-5}$M $E_2$ | 17,800 | NT* | NT* | - |
| Regular medium | 66,600 | 4,000 | 52,800 | 106 |
| Regular medium +1.4 x $10^{-5}$M TAM | 144,000 | 10,200 | 117,900 | 236 |

**\* Not Tested**

A novel TGF is provided, namely TGF-β2 which may be used to provide for growth regulating activity. The subject methods and compositions provide a means for preparing and using compositions which have anti-neoplastic activity. The compositions may also be used to detect cell lines responsive to not only the compositions themselves, but also compounds which induce synthesis of the compositions.

All publications and patent applications mentioned in this specification are indicative of the level of skill of those skilled in the art to which this invention pertains. All publications and patent applications are herein incorporated by reference to the same extent as if each individual publication or patent application was specifically and individually indicated to be incorporated by reference.

Although the foregoing invention has been described in some detail by way of illustration and example for purposes of clarity of understanding, it will be obvious that certain changes and modifications may be practiced within the scope of the appended claims.

## Claims

1. A method for inhibiting proliferation of neoplastic cells, said method comprising:

contacting said cells with a polypeptide comprising an amino acid sequence of at least eight amino acids included in the complete sequence:

A-L-D-A-A-Y-C-F-R-N-V-Q-D-N-C-C-L-R-P-L
Y-I-D-F-K-R-D-L-G-W-K-W-I-H-E-P-K-G-Y-N
A-N-F-C-A-G-A-C-P-Y-L-W-S-S-D-T-Q-H-S-R
V-L-S-L-Y-N-T-I-N-P-E-A-S-A-S-P-C-C-V-S
Q-D-L-E-P-L-T-I-L-Y-Y-I-G-K-T-P-K-I-E-Q
L-S-N-M-I-V-K-S-C-K-C-S;

wherein said polypeptide is characterized as being capable of inhibiting proliferation of neoplastic cells.

2. A method according to Claim 1, wherein said contacting is with at least one other anti-proliferative compound.

3. A method according to Claim 1, wherein said contacting is with a formulation comprising TGF-$\beta$2.

4. A method for inhibiting proliferation of neoplastic cells, said method comprising:

contacting said cells with an inducer of production of a polypeptide comprising an amino acid sequence of at least eight amino acids included in the complete sequence

A-L-D-A-A-Y-C-F-R-N-V-Q-D-N-C-C-L-R-P-L
Y-I-D-F-K-R-D-L-G-W-K-W-I-H-E-P-K-G-Y-N
A-N-F-C-A-G-A-C-P-Y-L-W-S-S-D-T-Q-H-S-R
V-L-S-L-Y-N-T-I-N-P-E-A-S-A-S-P-C-C-V-S
Q-D-L-E-P-L-T-I-L-Y-Y-I-G-K-T-P-K-I-E-Q
L-S-N-M-I-V-K-S-C-K-C-S;

wherein said polypeptide is characterized as being capable of inhibiting proliferation of neoplastic cells.

5. A method according to Claim 4, wherein said inducer is at least one of a compound with estrogenic activity, and a compound with anti-estrogenic activity.

6. A method according to Claim 5, wherein said inducer is 17$\beta$-estradiol and 1-p-$\beta$-dimethylaminoethoxyphenyl-trans-1,2-diphenylbut-1-ene.

7. A method for detecting responsiveness of a cell to an inducer of a polypeptide characterized as being capable of inhibiting proliferation of neoplastic cells and having an M$_r$ of about 24,000 by SDS-PAGE electrophoresis, and having an amino acid sequence which comprises:

A-L-D-A-A-Y-C-F-R-N-V-Q-D-N-C-C-L-R-P-L
Y-I-D-F-K-R-D-L-G-W-K-W-I-H-E-P-K-G-Y-N
A-N-F-C-A-G-A-C-P-Y-L-W-S-S-D-T-Q-H-S-R
V-L-S-L-Y-N-T-I-N-P-E-A-S-A-S-P-C-C-V-S
Q-D-L-E-P-L-T-I-L-Y-Y-I-G-K-T-P-K-I-E-Q
L-S-N-M-I-V-K-S-C-K-C-S;

said method comprising:

contacting said cell with an amount of said inducer sufficient to induce said polypeptide in an inducer-responsive cell;

collecting a sample of a fluid in contact with said cell;

combining said sample with an antibody which binds specifically to said polypeptide whereby a polypeptide-antibody complex is formed; and

detecting complex formation as indicative of responsiveness of said cell to said inducer.

8. A method according to Claim 7, wherein said inducer is at least one of a compound with estrogenic activity and a compound with anti-estrogenic activity.

9. A method according to Claim 8, wherein said compound having anti-estrogenic activity is 1-p-$\beta$-dimethylaminoethoxyphenyl-trans-1,2-diphenylbut-1-ene.

10. A substantially pure polypeptide composition having an amino acid sequence comprising:

A-L-D-A-A-Y-C-F-R-N-V-Q-D-N-C-C-L-R-P-L
Y-I-D-F-K-R-D-L-G-W-K-W-I-H-E-P-K-G-Y-N
A-N-F-C-A-G-A-C-P-Y-L-W-S-S-D-T-Q-H-S-R
V-L-S-L-Y-N-T-I-N-P-E-A-S-A-S-P-C-C-V-S
Q-D-L-E-P-L-T-I-L-Y-Y-I-G-K-T-P-K-I-E-Q
L-S-N-M-I-V-K-S-C-K-C-S;

wherein said polypeptide is characterized as being capable of inhibiting proliferation of neoplastic cells and having an M$_r$ of about 24,000 by SDS-PAGE electrophoresis.

11. A polypeptide according to Claim 10, wherein the specific activity of said polypeptide is at least 79 units/ng growth inhibitory activity.

12. A polypeptide according to Claim 11, wherein said polypeptide is obtained from inducer-supplemented medium conditioned by human tumor cells.

13. A polypeptide according to Claim 12, wherein said inducer is at least one of 17$\beta$-estradiol and 1-p-$\beta$-dimethylaminoethoxyphenyl-trans-1,2-diphenylbut-1-ene.

14. A polypeptide comprising an amino acid sequence of at least eight amino acids included in the complete sequence:

A-L-D-A-A-Y-C-F-R-N-V-Q-D-N-C-C-L-R-P-L
Y-I-D-F-K-R-D-L-G-W-K-W-I-H-E-P-K-G-Y-N
A-N-F-C-A-G-A-C-P-Y-L-W-S-S-D-T-Q-H-S-R
V-L-S-L-Y-N-T-I-N-P-E-A-S-A-S-P-C-C-V-S

14

Q-D-L-E-P-L-T-I-L-Y-Y-I-G-K-T-P-K-I-E-Q
L-S-N-M-I-V-K-S-C-K-C-S;
wherein said polypeptide is characterized as being capable of inhibiting proliferation of neoplastic calls.

15. A monoclonal antibody specific for a polypeptide composition comprising an amino acid sequence:
A-L-D-A-A-Y-C-F-R-N-V-Q-D-N-C-C-L-R-P-L
Y-I-D-F-K-R-D-L-G-W-K-W-I-H-E-P-K-G-Y-N
A-N-F-C-A-G-A-C-P-Y-L-W-S-S-D-T-Q-H-S-R
V-L-S-L-Y-N-T-I-N-P-E-A-S-A-S-P-C-C-V-S
Q-D-L-E-P-L-T-I-L-Y-Y-I-G-K-T-P-K-I-E-Q
L-S-N-M-I-V-K-S-C-K-C-S.

16. A monoclonal antibody as defined in claim 15, further comprising a label.

17. A monoclonal antibody according to Claim 15 or 16, wherein said monoclonal antibody is a human monoclonal antibody.

18. A method for producing a polypeptide comprising an amino acid sequence of at least eight amino acids included in the complete sequence
A-L-D-A-A-Y-C-F-R-N-V-Q-D-N-C-C-L-R-P-L
Y-I-D-F-K-R-D-L-G-W-K-W-I-H-E-P-K-G-Y-N
A-N-F-C-A-G-A-C-P-Y-L-W-S-S-D-T-Q-H-S-R
V-L-S-L-Y-N-T-I-N-P-E-A-S-A-S-P-C-C-V-S
Q-D-L-E-P-L-T-I-L-Y-Y-I-G-K-T-P-K-I-E-Q
L-S-N-M-I-V-K-S-C-K-C-S
which comprises:
introducing into a host cell an expression construct comprising a DNA sequence encoding said polypeptide under the regulatory control of transcriptional and translational regulatory signals functional in said host cell;
growing said host cell comprising said expression construct in a nutrient medium whereby said polypeptide is produced; and
isolating said polypeptide.

19. A method for preparing a polypeptide wherein said polypeptide is transforming growth factor beta-2 or a congener thereof and has an amino acid sequence substantially conforming with a fragment of at least eight amino acids of the following sequence
A-L-D-A-A-Y-C-F-R-N-V-Q-D-N-C-C-L-R-P-L
Y-I-D-F-K-R-D-L-G-W-K-W-I-H-E-P-K-G-Y-N
A-N-F-C-A-G-A-C-P-Y-L-W-S-S-D-T-Q-H-S-R
V-L-S-L-Y-N-T-I-N-P-E-A-S-A-S-P-C-C-V-S
Q-D-L-E-P-L-T-I-L-Y-Y-I-G-K-T-P-K-I-E-Q
L-S-N-M-I-V-K-S-C-K-C-S
said method comprising at least one of:
(a) synthesizing said polypeptide on a solid support; or
(b) isolating said polypeptide from inducer-supplemented medium conditioned by human tumor cells; or
(c) growing an expression host containing an expression construct comprising in the direction of transcription, a transcriptional regulatory region and a translational initiation region recognized by said host; a DNA sequence encoding said polypeptide, wherein the DNA sequence is a complete or partial structural gene isolated from natural sources, or a synthetic gene; and translational and transcriptional termination regions recognized by said host, wherein expression of said DNA sequence is under regulatory control of said initiation and termination regions, and wherein at least one of said initiation and termination regions is other than the natural region joined to the structural gene for transforming growth factor beta-2, when said DNA sequence is isolated from said natural sources.

20. A method according to Claim 19, wherein said polypeptide has said sequence.

21. A method according to Claim 19, wherein said polypeptide is isolated from inducer-supplemented medium conditioned by human tumor cells and said inducer is at least one of 17-beta-estradiol and 1-p-beta-dimethylaminoethoxyphenyl-trans-1,2-diphenylbut-1-ene.

22. A polypeptide as defined in Claims 19 or 20 when prepared by the method defined in Claims 19 or 20.

23. A DNA sequence comprising a gene encoding a polypeptide having an amino acid sequence comprising:

A-L-D-A-A-Y-C-F-R-N-V-Q-D-N-C-C-L-R-P-L
Y-I-D-F-K-R-D-L-G-W-K-W-I-H-E-P-K-G-Y-N
A-N-F-C-A-G-A-C-P-Y-L-W-S-S-D-T-Q-H-S-R
V-L-S-L-Y-N-T-I-N-P-E-A-S-A-S-P-C-C-V-S
Q-D-L-E-P-L-T-I-L-Y-Y-I-G-K-T-P-K-I-E-Q
L-S-N-M-I-V-K-S-C-K-C-S.

24. A DNA sequence encoding a transforming growth factor beta-2, said growth factor having the following amino acid sequence:

A-L-D-A-A-Y-C-F-R-N-V-Q-D-N-C-C-L-R-P-L
Y-I-D-F-K-R-D-L-G-W-K-W-I-H-E-P-K-G-Y-N
A-N-F-C-A-G-A-C-P-Y-L-W-S-S-D-T-Q-H-S-R
V-L-S-L-Y-N-T-I-N-P-E-A-S-A-S-P-C-C-V-S
Q-D-L-E-P-L-T-I-L-Y-Y-I-G-K-T-P-K-I-E-Q
L-S-N-M-I-V-K-S-C-K-C-S.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 200 341 (GENENTECH, INC.) <br> * whole document * <br><br> --- | 1-3,7, 10,14-20,22-24 | C 12 N 15/00 <br> A 61 K 37/00 <br> C 12 P 21/00 <br> C 07 K 7/00 |
| X | EP-A-0 105 014 (THE U.S.A. DEPARTMENT OF COMMERCE) <br> * page 13, lines 20-25; page 28, line 19 - page 29, line 4; claims 1,18,23-25,27,29 * <br><br> --- | 2,10,14 ,18-20, 22-24 | |
| P,X | EP-A-0 224 885 (WAKUNAGA SEIYAKU KABUSHIKI KAISHA) <br> * abstract; page 4, lines 8-16; page 5, lines 54-56; page 6, lines 26-36,55; page 38, table 15, example 23; claims 1-4,9 * <br><br> --- | 1-6 | |
| X | CHEMICAL ABSTRACTS, vol. 106, no. 15, 13th April 1987, page 130, column 1, abstract no. 114233r, Columbus, Ohio, US; R.B. DICKSON et al.: Activation of growth factor secretion in tumorigenic states of breast cancer induced by 17beta-estradiol or v-Ha-ras oncogene", & PROC. NATL. ACAD. SCI. U.S.A. 1987, 84(3), 837-841 <br><br> --- | 4-6,8, 12,21 | TECHNICAL FIELDS SEARCHED (Int. Cl.4) <br><br> C 12 N 15/00 <br> C 12 P 21/00 <br> A 61 K 37/00 |
| A | EP-A-0 132 021 (G. TODARO) <br> * abstract; page 14, lines 1-19; page 17, line 28 - page 18, line 6, page 24, lines 11-24; page 26, lines 13-26; claims 19,27,28,32 * <br><br> ----- | 7,10,14 ,15-17 | |

The present search report has been drawn up for all claims

| Place of search | Date of completion of the search | Examiner |
|---|---|---|
| BERLIN | 11-07-1988 | JULIA P. |

EPO FORM 1503 03.82 (P0401)